# EUROPEAN PATENT APPLICATION

(11) **EP 3 998 280 A1**
(43) Date of publication of application: **18.05.2022**
(21) Application number: 20837126.0
(22) Date of filing: 03.07.2020
(51) Int. Cl.: C07K 16/00, C12N 5/16, C12N 15/06, C12P 21/08

(54) **HYBRIDOMA AND METHOD FOR PRODUCING SAME, AND MONOCLONAL ANTIBODY AND METHOD FOR PRODUCING SAME**

(30) Priority: 08.07.2019 JP 2019127110
(71) Applicant: TERUMO Kabushiki Kaisha, Tokyo 151-0072 (JP)
(72) Inventor: URA, Takehiro, Nakakoma-gun, Yamanashi 409-3853 (JP)
(74) Representative: Haseltine Lake Kempner LLP
(86) International application number: PCT/JP2020/026218
(87) International publication number: WO 2021/006212

(57) **Abstract**

[Problem] An object of the present invention is to provide a means that makes it possible to obtain an antibody that specifically binds to the conformational epitope of a protein antigen and to produce a variety of monoclonal antibodies with unique specificity even in the case of conducting a booster.

[Solution] A non-human animal (animal for immunization) is immunized by intradermal administration of a protein antigen, antibody-producing cells are obtained from a regional lymph node corresponding to the administration site for the above antigen in the immunized animal, and a monoclonal antibody is made by hybridoma technology as described above.

## Description

### TECHNICAL FIELD

The present invention relates to a hybridoma and a method for making the same, a monoclonal antibody and a method for making the same.

### BACKGROUND ART

Monoclonal antibodies are highly sensitive detection probes that have high antigen-binding specificity and high binding strength. A monoclonal antibody is not only an essential research tool in life science in the post-genome era, but also a highly functional biological material that is commercially useful because it enables the mass production of antibodies of consistent quality. As a result, the market for antibody-based disease test agents, diagnostic agents, and pharmaceuticals has been continuing to expand.

There are two main methods of producing monoclonal antibodies as follows:
1) Hybridoma technology, in which a hybridoma is prepared by immunizing a non-human animal (animal for immunization) with an antigen; and
2) In vitro display technologies, in which a gene for the antigen-binding regions of an immunoglobulin is expressed through genetic engineering.

Although both technologies have their advantages and disadvantages, hybridoma technology has been implemented in many institutions because of its ease of system implementation. In the production of monoclonal antibodies by hybridoma technology, B-cells or a cell population containing B-cells collected from an animal immunized with a desired antigen are fused with myeloma cells, and from the resulting population of fused cells (hybridomas), a cell line (antibody-producing cells) producing antibodies that recognize the antigen is isolated. Traditionally, a spleen has been used as the origin of B cells, but in recent years, hybridoma production using lymphocytes derived from lymph nodes has become popular. Among them, the method using iliac nodes is considered to have excellent features, as it requires only one administration of antigen (immunization) and allows cell fusion operation to be performed in about two weeks after immunization. According to this method, for example, in rats, antigens are administered to the hindlimb footpad to rapidly enlarge the iliac nodes, which can then be used as material for hybridoma preparation (Japanese Patent Laid-Open No. H06-209769).

Here, the immune response of the animal is greatly influenced by the nature of the individual used (species, sex, age, health status). In addition, some antigens are less effective in eliciting an immune response in individuals, and often do not cause sufficient lymph node enlargement in immunized individuals. Therefore, in order to stably produce monoclonal antibodies, it is necessary to establish a technique to reproducibly obtain B-cells that have acquired the antibody-producing ability. As a means to solve such a problem, Japanese Patent Laid-Open No. 2009-284771 proposes a method for making a monoclonal antibody, including making a hybridoma producing a monoclonal antibody capable of recognizing an antigen by using enlarged lymph nodes as a B-cell source, which are obtained by administering the antigen to the neck or back of mice.

### SUMMARY OF INVENTION

### Technical Problem

In the production of monoclonal antibodies by hybridoma technology, it is generally difficult to obtain antibodies that recognize the conformation of an antigen protein (i.e., that bind specifically to a conformational epitope). According to the inventor's study, it has been confirmed that even with the technologies described in Japanese Patent Laid-Open No. H06-209769 and Japanese Patent Laid-Open No. 2009-284771, it is still difficult to solve this problem. If an antibody that specifically binds to the conformational epitope of a protein antigen can be obtained, it will contribute to the development of disease test agents, diagnostic agents, pharmaceuticals, etc. based on a different action mechanism from an antibody that binds to linear epitopes (sequential epitopes).

In addition, the advantage of the conventional hybridoma technology is that antibodies with high affinity to an antigen can be selectively obtained through multiple antigen administrations by a booster shot. On the other hand, there is a problem that the diversity of antibodies obtained is lost as booster shots are conducted.

It is therefore an object of the present invention to provide a means that makes it possible to obtain an antibody that specifically binds to the conformational epitope of a protein antigen and to produce a variety of monoclonal antibodies with unique specificity even in the case of conducting a booster shot.

### Means for Solving Problem

The present inventors have conducted intensive studies to solve the above-described problems. As a result, it has been surprisingly found that, when a non-human animal (animal for immunization) is immunized by intradermal administration of a protein antigen, antibody-producing cells are obtained from a regional lymph node corresponding to the administration site for the above antigen in the immunized animal, and a monoclonal antibody is produced by hybridoma technology as described above, the production of an antibody that specifically binds to the conformational epitope of the above antigen is facilitated, which has led the completion of the present invention.

In other words, according to one aspect of the present invention, there are provided a method for making a hybridoma producing a monoclonal antibody that specifically recognizes a conformational epitope of a protein antigen, the method including: immunizing a non-human animal by intradermal administration of the protein antigen to the non-human animal; collecting antibody-producing cells from a regional lymph node which corresponds to an administration site for the protein antigen in the non-human animal immunized by the administration of the protein antigen; fusing the collected antibody-producing cells with myeloma cells to prepare a hybridoma population; and selecting, from the hybridoma population, a specific hybridoma producing a monoclonal antibody that specifically recognizes a conformational epitope of the protein antigen, and a hybridoma made by the making method.

According to another aspect of the present invention, there are provided a method for making a monoclonal antibody, the method including: obtaining a specific hybridoma by the method for making a hybridoma according to the above-described aspect of the present invention; and obtaining a monoclonal antibody produced by the obtained specific hybridoma, and a monoclonal antibody made by the making method.

### BRIEF DESCRIPTION OF DRAWINGS

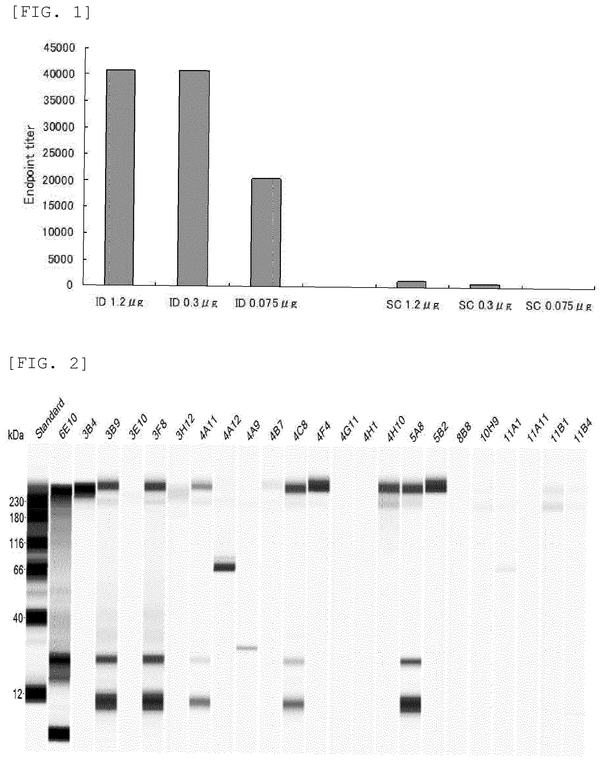

FIG. 1 is a graph that compares antibody titers in the blood after administering different amounts of antigen (Japanese encephalitis vaccine) into the back of mice through intradermal administration or subcutaneous injection in Examples.
FIG 2 is a gel electrophoresis photograph showing the result of Western blotting through which binding of a monoclonal antibody made by the making method (intradermal administration) according to the present invention and a monoclonal antibody produced in the same manner through subcutaneous injection to Aβ1-42 peptide (monomer) and protofibril is evaluated in Examples.

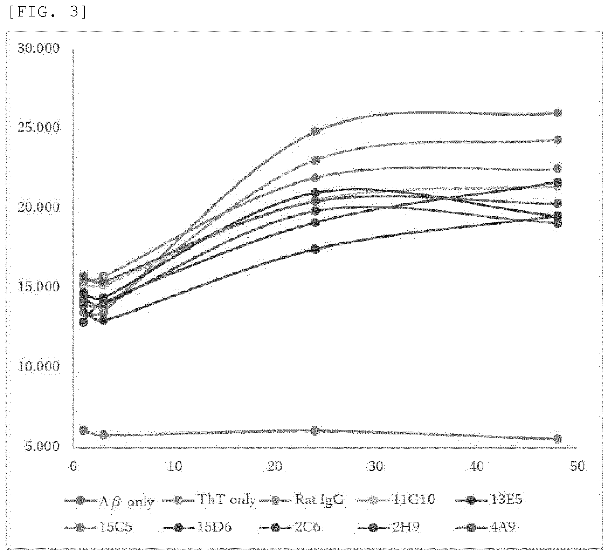

FIG 3 is a graph showing the result of determining the presence or absence of competitive inhibition on an epitope binding site using a fluorescent dye for seven monoclonal antibodies out of the monoclonal antibody made by the making method (intradermal administration) according to the present invention and the monoclonal antibody produced in the same manner through subcutaneous injection that were shown to inhibit polymerization of Aβ peptide in Examples.

### DESCRIPTION OF EMBODIMENTS

Hereinafter, an embodiment of the present invention will be described with reference to the attached drawings.

### «Method for Making Hybridoma»

An aspect of the present invention relates to a method for making a hybridoma producing a monoclonal antibody that specifically recognizes a conformational epitope of a protein antigen, the method including: immunizing a non-human animal by intradermal administration of the protein antigen to the non-human animal; collecting antibody-producing cells from a regional lymph node which corresponds to an administration site for the protein antigen in the non-human animal immunized by the administration of the protein antigen; fusing the collected antibody-producing cells with myeloma cells to prepare a hybridoma population; and selecting from the hybridoma population a specific hybridoma producing a monoclonal antibody that specifically recognizes a conformational epitope of the protein antigen. The method for making a hybridoma according to the present invention can obtain an antibody that specifically binds to the conformational epitope of a protein antigen and produce a variety of monoclonal antibodies with unique specificity even in the case of conducting a booster shot.

Regarding conventional methods for producing a monoclonal antibody by hybridoma technology using an immunized animal, there have been reports on, for example, methods in which the antibody is obtained efficiently with increased production efficiency, and methods in which the antibody with high affinity is obtained. In contrast, for the variety of monoclonal antibodies obtained, there was no clear way other than to limit the animal species.

In the conventional method for making monoclonal antibodies by hybridoma technology, an organ is removed from an animal that has produced sufficient antibodies through multiple antigen administrations by a booster shot. This booster shot has the advantage of selectively obtaining antibodies with high affinity to the antigen. However, there was a problem that obtaining antibodies with a rich diversity was conversely difficult. Thus, in order to obtain diverse clones from an immunized animal, techniques of reducing the number of immunizations by devising the antigen and of increasing the production efficiency when establishing a clone have been studied.

In contrast to the situation of the prior art as described above, the present invention can solve the problem in the prior art by a simple technique in which hybridomas are prepared using lymphocytes collected from a regional lymph node for an administration site with intradermal administration taken as an administration route at the time of antigen administration.

In general, intradermal administration of antigens is known to improve immunogenicity, increase specific antibody titers in the blood at an early stage, and have a high antibody induction capacity. Therefore, utilizing such characteristics of intradermal administration, technical studies are underway on vaccine administration routes, for example. On the other hand, when these characteristics of intradermal administration are considered in light of a method for making a monoclonal antibody, it is thought that a monoclonal antibody of interest can be established early by reducing the number of immunizations. That is, the method for making a monoclonal antibody according to the present invention has the potential to improve antigenicity in an immunized animal, and thus has a feature of having a high possibility of obtaining a variety of monoclonal cells from a lymphocyte source by reducing the number of immunizations.

However, the present inventors dared to perform intradermal administration of the antigen multiple times and examined its characteristics at a single cell level. As a result, they found that the fact that it is possible to establish a rich variety of monoclonal antibodies despite multiple long-term immunizations.

Hereinafter, the method for making a hybridoma according to the present invention and the method for making a monoclonal antibody using the hybridoma will be described in detail in the order of the process.

### (Immunization through intradermal administration of a protein antigen)

First, a protein antigen is prepared. The protein antigen may be any protein antigen that maintains its native conformation and is not particularly limited. The protein antigen may be a full-length protein or a partial protein containing a target site that can be an epitope. Examples of the protein antigen include amyloid aggregates of proteins and peptides (e.g., amyloid, α-synuclein, β2-microglobulin, and prion), amorphous aggregates of proteins and peptides (e.g., inclusion bodies of proteins expressed in E. coli), G protein-coupled receptors with transmembrane domains, ionotropic receptors, and receptor tyrosine kinase. Viral antigens, inactivated viral antigens, bacterial antigens, toxin antigens, infection vaccines, cell lysates, peptides, etc. may also be used. In addition to these, CD antigens, cell adhesion molecules, tumor antigens, cytokines, growth factors, proliferators, nutritional factors, etc. may be used. The protein antigen may be made of a recombinant protein derived from plasmid DNA, take the form of a multimer such as a dimer or trimer, or be a precursor that can form a multimer in vivo. The protein antigen is preferably an amyloid aggregate or amorphous aggregate of proteins or peptides, or a protein including a transmembrane domain, more preferably an amyloid aggregate of proteins or peptides, and is particularly preferred to be an aggregate of amyloid β protein. In a preferred embodiment, the protein antigen that can form a multimer or an aggregate is intradermally administered to the above non-human animal in the form of a multimer or an aggregate. In another embodiment, the protein antigen may be intradermally administered by DNA immunization, or the protein antigen may be intradermally administered to the above non-human animal in the form of recombinant cells expressing the protein antigen.

From another point of view, the protein antigen is preferably a protein antigen with weak immunogenicity. Examples of such a protein antigen with weak immunogenicity include antigens with high homology to animals for immunization, glycosylated antigens, and antigens with a low molecular weight (e.g., a molecular weight of less than 6000) . For example, when using a low-molecular-weight peptide as an antigen, it has been widely practiced to have the peptide binding to a carrier protein such as KLH and OVA to form a composite with a high molecular weight, and then use the composite as an antigen for immunization. However, most of the monoclonal antibodies obtained by such a method specifically recognize a carrier protein rather than the target low-molecular-weight peptide, and thus, the antibody of interest may not be obtained in some cases. Even in such a case, use of the hybridoma and monoclonal antibody production methods according to the present aspect can increase the possibility of obtaining a monoclonal antibody that specifically recognizes the target low-molecular-weight peptide rather than the carrier protein, and thus is preferable.

On the other hand, a non-human animal is prepared as an animal for immunization. For the non-human animal, any animal other than humans can be used without restriction; however, it is known that the affinity of antibodies depends on the type of animal species for immunization, and when the antigen affinity of the monoclonal antibody of interest is important, an appropriate animal species for immunization should be selected. Also, a target molecule derived from a living body may be recognized as an autoantigen for some animal species, and thus, there may be restrictions due to animals for immunization, such as selecting animal species with a low peptide sequence homology. The non-human animal used as the animal for immunization is preferably a warm-blooded animal. Examples of the warm-blooded animal to be used include mammals (e.g., a rat, a mouse, a rabbit, a guinea pig, a hamster, a cynomolgus monkey, a dog, sheep, a goat, a donkey, a pig) and birds (e.g., a chicken) . Among them, mammals are more preferable, rodents (e.g., a rat, a mouse, a rabbit, a guinea pig, a hamster) are even more preferable, a rat, a mouse, and a rabbit are especially preferable, and a rat is most preferable. In order to avoid the problem of anti-Ig antibody production, it is preferable to use mammals of the same species as the target of administration, but mice and rats are generally preferably used for monoclonal antibody preparation. Note that genetically modified animals, such as transgenic animals, may be employed as the non-human animal for the animal for immunization.

The hybridoma and monoclonal antibody production methods according to the present aspect has a feature that the administration of the protein antigen is conducted through intradermal administration. As will be clearly demonstrated in the section of Examples below, immunizing an animal for immunization by administering a protein antigen through intradermal administration attains an advantage that it is possible to obtain an antibody that specifically binds to the conformational epitope of the protein antigen and to produce a variety of monoclonal antibodies with unique specificity even in the case of conducting a booster shot.

There are no particular restrictions on a specific manner of the intradermal administration, and the operation itself of the intradermal administration can be carried out with reference to conventional known knowledge. In addition, there are no particular restrictions on the administration site for the intradermal administration of the protein antigen; however, from the point of view that the skin is thick and intradermal administration is easily performed, it is preferable to use the back or auricle of the animal for immunization (the aforementioned non-human animal) as an administration site, and it is especially preferable to use the back. Note that the depth of intradermal administration is also difficult to uniquely define because the thickness of the skin varies depending on the animal species and the administration site, but as an example, intradermal administration at a depth of 0.1 to 3.0 mm from the epidermis is preferable. From the point of view of effective use of the protein antigen to be administered, it is preferable to administer the protein antigen only intradermally (i.e., only into a combination of the epidermal and dermal regions) . The skin of a mouse is relatively thin, about 0.2 mm thick, while that of a pig with thick skin is about 3.0 mm thick. Although the intradermal administration of the protein antigen may be performed at multiple snites, it is preferable to administer the protein antigen at a single site from the point of view of reliable administration without leakage.

A protein antigen is usually administered by administering a solution with the antigen dissolved therein; and there are no restrictions on the solvent used in this process, and water such as water for injection or purified water, as well as conventionally known buffer solutions may be used as the solvent. The protein antigen may be administered intradermally to the non-human animal by itself or may be administered with a carrier or diluent. In addition, Freund's complete adjuvant or Freund's incomplete adjuvant may be administered to enhance the antibody-producing ability at the time of the administration. The administration is usually performed once every 1 to 6 weeks, for a total of about 2 to 10 times. Here, the hybridoma and monoclonal antibody production methods according to the present invention have the advantage that the diversity of the antibodies obtained is less likely to be impaired even when a booster shot is performed, unlike the conventionally known production methods. Therefore, in a preferred embodiment of the present invention, a booster shot is performed (that is, intradermal administration of the protein antigen is performed multiple times). In a more preferred embodiment, the protein antigen is administered three or more times, and even more preferably four or more times.

When the protein antigen is to be administered, there are no restrictions on the dose volume (e.g., in the above solution) as long as the protein antigen can be administered intradermally without fail. From the point of view of effective use of the administered protein antigen, a smaller dose volume is preferable, e.g., 20 µL or less. However, when the skin at the administration site in the animal for immunization is thick, a relatively large dose volume can be tolerated. From this point of view, in a preferred embodiment in which a solution containing the protein antigen is intradermally administered, a single dose volume of the above solution is preferably 100 µL or less per unit thickness [mm] of the skin at the administration site.

### (Collection of antibody-producing cells)

The preparation of a monoclonal antibody according to the present aspect also has a feature that an individual with an increased antibody titer is selected from non-human animals immunized with a protein antigen, and the regional lymph node for the administration site is taken out, for example, 2 to 5 days after the final immunization to isolate antibody-producing cells. Thus, the combination of the intradermal administration of the protein antigen and isolation of antibody-producing cells from the regional lymph node can deliver the advantageous effect of the present invention described above. Note that there are no particular restrictions on the specific type of the regional lymph node for the administration site, and it should be determined according to the administration site of the protein antigen. Examples of a lymph node that can be the regional lymph node include axillary lymph nodes, brachial lymph nodes, submandibular lymph nodes, parotid lymph nodes, and inguinal lymph nodes. Depending on the administration site, antibody-producing cells may be collected from not only one regional lymph node but also a plurality of regional lymph nodes.

### (Preparation and section of a hybridoma)

The antibody-producing cells collected from the regional lymph node of the immunized animal as described above are used for cell fusion with myeloma (bone marrow tumor) cells, whereby a hybridoma population producing antibodies can be prepared. In this case, an antibody titer in the serum can be measured, for example, by having a labelled antigen reacting with antiserum and then measuring the activity of a labelling agent bound to the antibody.

There are no particular restrictions on the myeloma cells as long as they can produce hybridomas that secrete a large amount of antibodies, but myeloma cells that do not themselves produce or secrete antibodies are preferable, and myeloma cells with high cell fusion efficiency are more preferable. To facilitate the selection of the hybridoma, it is preferable to use an HAT (hypoxanthine-aminopterin-thymidine)-sensitive strain. Examples of mouse myeloma cells include NS-1, P3U1, SP2/0, and AP-1; examples of rat myeloma cells include R210.RCY3, and Y3-Ag1.2.3; and examples of human myeloma cells include SKO-007, GM1500-6TG-2, LICR-LON-HMy2, and UC729-6.

The operation of the cell fusion can be carried out in accordance with known methods, e.g., the method of KOHLER andMILSTEIN [Nature, 256, 495 (1975)] . Examples of a fusion promoter include polyethylene glycol (PEG) and Murine respirovirus, but PEG is preferably used. There is no particular restriction on the molecular weight of PEG, but any one of from PEG 1000 to PEG 6000 is preferred for its low toxicity and relatively low viscosity. A concentration of PEG is, for example, about 10 to 80%, preferably about 30 to 50%. For a solution for dilution of PEG, various buffers such as serum-free culture medium (e.g., RPMI 1640), a complete culture medium containing about 5 to 20% serum, phosphate-buffered saline (PBS), and Tris buffer can be used. If desired, DMSO (e.g., about 10 to 20%) can be added. A pH of a fusion solution is, for example, about 4 to 10, preferably about 6 to 8.

The preferred ratio of the number of antibody-producing cells to the number of myeloma cells is usually about 1:1 to 20:1, and cell fusion can be efficiently performed by incubating (e.g., performing CO₂ incubation on) the cells usually at 20 to 40°C, preferably at 30 to 37°C, usually for 1 to 10 minutes.

The antibody-producing cell line can also be obtained by infecting the antibody-producing cell with a virus that can transform the lymphocyte and immortalize the cell. Examples of such a virus include Epstein-Barr (EB) virus. Most people have been infected with this virus as an asymptomatic infection of infectious mononucleosis and thus are immune to this virus; however, when ordinary EB virus is used, appropriate purification should be performed because viral particles are also produced. As an EB system without the possibility of viral contamination, it is also preferable to use a recombinant EB virus that retains the ability to immortalize the β-lymphocyte but lacks the ability to replicate viral particles (e.g., a deletion of a switch gene for the transition from a latent infection state to a lytic infection state).

Human antibody-secreting cells that have acquired the infinite proliferation ability by undergoing transformation can be back-fused with mouse or human myeloma cells in order to stably maintain the antibody-secreting ability. For the myeloma cells, the same as described above can be used.

Then, from the hybridoma population prepared above, a hybridoma (herein referred to simply as "a specific hybridoma") is selected which produces a monoclonal antibody specifically recognizing the conformational epitope of the protein antigen administered for immunization. By using the hybridoma thus selected, the desired monoclonal antibody (which specifically recognizes a conformational epitope) can be produced.

Screening and breeding of hybridomas is usually performed in an animal cell culture medium (e.g., RPMI1640) containing 5 to 20% FCS or a serum-free culture medium having a cell growth factor, with HAT (hypoxanthine, aminopterin, thymidine) added. Hypoxanthine, aminopterin, and thymidine have concentrations of, for example, about 0.1 mM, about 0.4 µM, and about 0.016 mM, respectively. Ouabain resistance can be used in the selection of human-mouse hybridomas. Since a human cell line is more sensitive to ouabain than a mouse cell line, unfused human cells can be eliminated by adding ouabain to the culture medium at about 10⁻⁷ to 10⁻³ M.

Feeder cells or a certain cell culture supernatant is preferably used for the hybridoma selection. As the feeder cells, there can be used a heterogeneous cell strain with a limited survival time which helps the emergence of hybridomas and dying out of its own, cells which can produce a large amount of a growth factor useful for the emergence of hybridomas but have a reduced proliferative capacity due to irradiation, and the like. Examples of a mouse feeder cell include spleen cells, macrophages, blood, and thymocytes; and examples of a human feeder cell include peripheral blood monocytes. Examples of the cell culture supernatant include primary culture supernatants for the aforementioned various cells and culture supernatants for various established cell lines.

The hybridomas can also be selected by fluorescence-labelling the antigen and reacting it with the fused cells, and then separating cells binding the antigen using a fluorescence-activated cell sorter (FACS) . In this case, hybridomas producing antibodies against the target antigen can be directly selected, which can greatly reduce the cloning effort.

A variety of methods can be used to clone hybridomas producing monoclonal antibodies against a target antigen.

Since aminopterin inhibits many cell functions, it is preferable to remove it from the culture medium as soon as possible. In the case of mice and rats, most myeloma cells die within 10 to 14 days, and thus, aminopterin can be removed from 2 weeks after fusion. However, human hybridomas are usually maintained in an aminopterin-containing culture medium for about 4 to 6 weeks after fusion. It is desirable that hypoxanthine and thymidine be removed at least one week after the removal of aminopterin. That is, in the case of mouse cells, for example, after 7 to 10 days of fusion, a hypoxanthine and thymidine (HT) -containing complete culture medium (e.g., RPMI 1640 with addition of 10% FCS) is added or replaced. Visible clones appear about 8 to 14 days after fusion. When the diameter of the clone is about 1 mm, it is possible to measure the amount of antibodies in the culture supernatant.

The amount of antibodies can be measured, for example, by: a method in which a hybridoma culture supernatant is added to a solid phase (e.g., a microplate) on which a target antigen or derivative thereof, or a partial peptide thereof (including a partial amino acid sequence used as the antigenic determinant) is adsorbed directly or with a carrier, then, anti-immunoglobulin (IgG) antibodies (antibodies against IgG derived from animals of the same species as the animal from which the original antibody-producing cell is derived are used) or protein A labelled with radioactive substances (e.g., ¹²⁵I, ¹³¹I, ³H, ¹⁴C), enzymes (e.g., β-galactosidase, β-glucosidase, alkaline phosphatase, peroxidase, malate dehydrogenase), fluorescent substances (e.g., fluorescamine, fluorescein isothiocyanate), or luminescent substances (e.g., luminol, luminol derivatives, luciferin, lucigenin) are added, and antibodies against the target antigen (antigenic determinant) bound to the solid phase are detected; or a method in which a hybridoma culture supernatant is added to a solid phase on which an anti-IgG antibody or protein A is adsorbed, a target antigen or derivative thereof, or a partial peptide thereof that is labelled with the same labelling agent as above is added, and antibodies against the target antigen (antigenic determinant) bound to the solid phase are detected.

Although limiting dilution analysis is usually used as a cloning method, cloning using soft agar or cloning using FACS (as described above) is also possible. Cloning through limiting dilution analysis can be performed by, for example, but not limited to, the following procedure.

The amount of antibodies is measured as described above to select positive wells. Appropriate feeder cells are selected and added to a 96-well plate. Cells are aspirated from antibody-positive wells and suspended at a density of 30 cells/mL in a complete culture medium (e.g., RMPI1640 with 10% FCS and P/S added), 0.1mL (3 cells/well) of the suspension is added to the well plate with the feeder cells added, the remaining cell suspension is diluted to 10 cells/mL and spread in another well (1 cell/well), and the rest of the cell suspension is further diluted to 3 cells/mL and spread in still another well (0.3 cell/well) . Culture is performed for about 2 to 3 weeks until visible clones appear, the amount of antibodies is measured, positive wells are selected, and cloning is performed again. Since cloning is relatively difficult in the case of human cells, a plate with 10 cells/well should also be prepared. Usually, a monoclonal antibody-producing hybridomas can be obtained by subcloning twice; however, it is desirable to perform additional re-cloning periodically for several months to confirm the stability of the hybridomas.

The hybridomas can be cultured in vitro or in vivo. Examples of a method for the in vitro culture include a method in which the monoclonal antibody-producing hybridomas obtained as described above are cultured with gradual scale-up starting from well plates while maintaining the cell density, for example, at about 10⁵ to 10⁶ cells/mL and gradually reducing the FCS concentration.

Examples of a method for the in vivo culture include a method in which a mouse having plasmacytoma (MOPC) induced by intraperitoneal injection of mineral oil (a mouse histocompatible with the parent line of the hybridomas) is injected intraperitoneally with the hybridomas by about 10⁶ to 10⁷ cells after 5 to 10 days, and its ascites is taken out under anesthesia 2 to 5 weeks later.

Determination as to whether a given hybridoma is a specific hybridoma producing a monoclonal antibody that specifically recognizes the conformational epitope of a protein antigen can be made by evaluating the reactivity of the monoclonal antibodies produced by the hybridoma against aggregates of the above protein antigen (e.g., protofibrils, which are aggregates of Aβ peptide) . In this case, the above evaluation can be performed with higher accuracy by evaluating the reactivity relative to the reactivity against the monomer of the above protein antigen or the peptide with the conformation of the above protein antigen disrupted. If the protein antigen is one that can form a multimer or aggregate, by using the gel that is electrophoresed so that the multimer or aggregate is arranged in order of molecular weight to perform Western blotting with the monoclonal antibody to be evaluated, it is possible to determine whether the epitope specifically recognized by the monoclonal antibody is a conformational epitope based on the size of the molecules bound by the monoclonal antibody to be evaluated.

### (Purification of a monoclonal antibody)

Separation and purification of the monoclonal antibodies can be performed in accordance with known methods, such as immunoglobulin separation and purification methods (e.g., salting-out, alcohol precipitation, isoelectric precipitation, electrophoresis, adsorption/desorption with ion exchangers (e.g., DEAE, QEAE), ultracentrifugation, gel filtration, a specific purification method in which only an antibody is recovered using an antigen-binding solid phase or an active adsorbent such as protein A or protein G, and the binding is dissociated to obtain the antibody).

In the manner described above, the hybridomas are cultured in vivo or in vitro in an immunized animal, and the antibody is taken from its fluid or culture, whereby the monoclonal antibody can be produced.

Although there are no particular restrictions on specifics of an affinity of the resulting monoclonal antibody for the protein antigen, in a preferred embodiment, the monoclonal antibody has an equilibrium dissociation constant of 1 × 10⁻⁷ [M] or less, and more preferably 1 × 10⁻⁹ or less [M] with respect to the conformational epitope.

In a preferred embodiment, a monoclonal antibody made by the method for making a monoclonal antibody according to the present invention is used as a pharmaceutical product for administration to humans. For this reason, the monoclonal antibody according to the present invention is preferably an antibody with a reduced risk of antigenicity when administered to humans (specifically, a fully human antibody, a humanized antibody, a mouse-human chimeric antibody, etc.), and particularly preferably a fully human antibody. The humanized antibody and the chimeric antibody can be prepared in a genetically engineered manner according to the methods described below. Although the fully human antibody can be produced from the human-human (or mouse) hybridomas described above, it is desirable to use a human antibody-producing animal (e.g., mouse) or phage display technology as described below in order to provide large amounts of antibodies in a stable and low-cost manner.

### (i) Preparation of a chimeric antibody

The term "chimeric antibody" herein refers to an antibody in which the sequences of the variable regions (V_{H} and V_{L}) of the H and L chains are derived from one mammalian species and the sequences of the constant regions (C_{H} and C_{L}) are derived from another mammalian species. It is preferable that the sequence of the variable region be derived from an animal species that can easily produce hybridomas, such as a mouse, and the sequence of the constant region be derived from a mammalian species that will be the target of administration.

Examples of the method for preparing a chimeric antibody include the method described in U.S. Patent No. 6,331,415 and a partially modified version thereof. More specifically, first, mRNA or total RNA is prepared from the monoclonal antibody-producing hybridoma (e.g., mouse-mouse hybridoma) obtained as described above to synthesize cDNA according to conventional methods. Then, using the cDNA as a template, the DNA encoding V_{H} and V_{L} is amplified by PCR according to conventional methods using appropriate primers (e.g., oligo DNA containing the nucleic acid sequences encoding the N-terminal sequences of V_{H} and V_{L} as sense primers and oligo DNA that hybridizes with the nucleic acid sequences encoding the N-terminal sequences of C_{H} and C_{L} as antisense primers (see Bio/Technology, 9:88-89,1991, for example)), and purified. By the same method, DNA encoding C_{H} and C_{L} is amplified by RT-PCR from RNA prepared from a lymphocyte, etc. of another mammalian (e.g., human), and purified. V_{H} is linked with C_{H}, and V_{L} is linked with C_{L} using a conventional method, and the resulting chimeric H-chain DNA and chimeric L-chain DNA are inserted into their respective appropriate expression vectors (e.g., a vector containing a promoter that is transcriptionally active in CHO cells, COS cells, mouse myeloma cells, etc. (e.g., CMVpromoter, SV40 promoter) . DNA encoding both strands may be inserted into separate vectors or tandemly into a single vector. The host cell is transformed with the resulting chimeric H chain- and chimeric L chain-expression vectors. Examples of the host cell include animal cells, such as mouse myeloma cells mentioned above, as well as Chinese hamster ovary (CHO) cells, COS-7 cells derived from monkeys, Vero cells, and GHS cells derived from rats. For the transformation, any method that can be applied to animal cells may be used, but preferably an electroporation technique is used. After culturing in a culture medium suitable for the host cells for a certain period of time, the culture supernatant is collected and purified in the same manner as described above, whereby the chimeric monoclonal antibody can be isolated. Alternatively, using, as the host cell, germline cells of animals such as cattle, goats, and chickens, for which transgenic technology has been established and knowledge for mass reproduction as livestock (poultry) has been accumulated, a transgenic animal can be prepared by a conventional method, whereby chimeric monoclonal antibodies can be obtained easily and in large quantities from the milk or eggs of the resulting animal. In addition, by using, as the host cell, plant cells for which transgenic technology has been established and which has been grown in large quantities as major crops, such as corn, rice, wheat, soybean, and tobacco, a transgenic plant can be prepared by applying microinjection and electroporation to protoplasts, a particle gun technique and a Ti vector technique to intact cells, etc., whereby chimeric monoclonal antibodies can be obtained in large quantities from the resulting seeds and leaves.

The resulting chimeric monoclonal antibody can be digested by papain to yield Fab, and by pepsin to yield F (ab')2.

In addition, DNA encoding mouse V_{H} and V_{L} can be linked with an appropriate linker, such as a peptide having, for example, 1 to 40 amino acids, preferably 3 to 30 amino acids, more preferably 5 to 20 amino acids (e.g., [Ser-(Gly)m]n or [(Gly)m-Ser]n, where m is an integer from 0 to 10 and n is an integer from 1 to 5) to form a scFv; DNA encoding CH3 can be linked with an appropriate linker to form a minibody monomer; or DNA encoding full-length C_{H} can be linked with an appropriate linker to form a scFv-Fc monomer. The DNA encoding such antibody molecules that are modified (conjugated) in a genetically engineered manner can be expressed in a microorganism such as E. coli and yeast under the control of an appropriate promoter, and the antibody molecules can be produced in large quantities.

When DNA encoding mouse V_{H} and V_{L} is inserted in a tandem manner downstream of a single promoter and introduced into E. coli, a dimer called Fv is formed by monocistronic gene expression. When an appropriate amino acid in FR of V_{H} and V_{L} is replaced with Cys using molecular modeling, a dimer called dsFv is formed by an intermolecular disulfide bond between the two chains.

### (ii) Humanized antibody

The term "humanized antibody" herein refers to an antibody in which the sequences of all regions (i.e., the constant region and the framework region (FR) in the variable region) except the complementarity-determining region (CDR) in the variable region are derived from a human, and only the CDR sequence is derived from another mammalian species. For the other mammalian species, an animal species that can easily produce hybridomas, such as a mouse, is preferable.

Examples of the method for preparing the humanized antibody include the methods described in U.S. Patent No. 5,225,539, U.S. Patent No. 5,585,089, U.S. Patent No. 5,693,761, and U.S. Patent No. 5,693,762, and partially modified versions thereof. More specifically, in the same way as in the case of the above chimeric antibody, DNA encoding V_{H} and V_{L} derived from mammalian species other than human (e.g., mouse) is isolated, and then sequenced using an automated DNA sequencer (for example, manufactured by Applied Biosystems) according to a conventional method, and the resulting nucleic acid sequence or the amino acid sequence inferred therefrom is analyzed using a known antibody sequence database (e.g., Kabat database (see Kabat et al., "Sequences of Proteins of Immunological Interest," US Department of Health and Human Services, Public Health Service, NIH publication, 5th edition, 1991), etc.) to determine the CDR and FR of both chains. A nucleic acid sequence in which the CDR coding region of the nucleic acid sequence encoding the L and H chains of a human antibody with an FR sequence similar to a determined FR sequence (e.g., human kappa-type L chain subgroup I and human H chain subgroup II or III (see Kabat et al., 1991 (mentioned above)) is replaced with a nucleic acid sequence encoding a determined heterologous CDR is designed, and then the nucleic acid sequence is divided into fragments of about 20 to 40 bases, and further, a sequence complementary to the nucleic acid sequence is divided into fragments of about 20 to 40 bases so that they overlap alternately with the former fragments. Each fragment can be synthesized using a DNA synthesizer, and then hybridized and ligated according to a conventional method to construct DNA encoding V_{H} and V_{L} having FR derived from human and CDR derived from other mammalian species . For faster and more efficient transfer of the CDR derived from other mammalian species to the human-derived V_{H} and V_{L}, use of a PCR site-directed mutagenesis is preferred. Examples of such a method include the successive CDR transfer method described in JP Japanese Patent Laid-Open No. H5-227970. The DNAs encoding V_{H} and V_{L} thus obtained can be linked respectively to the DNAs encoding human-derived C_{H} and C_{L} in the same manner as in the case of the chimeric antibody described above, and then introduced into an appropriate host cell, thereby cells that produce a humanized antibody or transgenic plant or animal can be produced.

The humanized antibody, similarly to the chimeric antibody, can be modified into scFv, scFv-Fc, minibody, dsFv, Fv, etc. using a genetic engineering technique, and can be produced in microorganisms such as E. coli and yeast using an appropriate promoter.

The technology for preparing the humanized antibody can also be applied to, for example, preparation of a monoclonal antibody that can be preferably administered to another animal species for which technology for preparing hybridomas has not yet been established. For example, an animal that is widely bred as livestock (poultry), such as cows, pigs, sheep, goats, and chickens, or a pet animal such as dogs and cats can be taken as a target.

### (iii) Preparation of a fully human antibody using a human antibody-producing animal

If a functional human Ig gene is introduced into a non-human animal with its endogenous immunoglobulin (Ig) gene knocked out (KO), and the animal is immunized with an antigen, the human antibody will be produced instead of the antibody derived from the animal. Therefore, it is possible to obtain a fully human monoclonal antibody by the same method as the conventional method for preparing a mouse monoclonal antibody by using an animal for which hybridoma production technology has been established, such as a mouse. First, some human monoclonal antibodies produced using human antibody-producing mice obtained by crossing mice in which the H- and L-chain minigenes of human Ig were introduced using conventional transgenic (Tg) techniques with mice in which the endogenous mouse Ig gene was inactivated using conventional KO techniques (see Immunol. Today, 17, 391-397 (1996)) are already in the clinical stage, and to date, the production of anti-human Ig human antibodies (HAHA) has not been reported.

Subsequently, Abgenix, Inc. (product name: XenoMouse (Nat. Genet.,15,146-156 (1997); see U.S. Patent No. 5,939,598, etc.)) and Medarex (product name: Hu-Mab Mouse (Nat. Biotechnol., 14,845-851 (1996); see U.S. Patent No. 5, 545,806, etc.)) have prepared Tg mice with larger human Ig genes introduced using yeast artificial chromosome (YAC) vectors, which can produce a wider repertoire of human antibodies. However, in the case of the H chain, for example, the diversity of the human Ig gene is achieved by the VDJ exon, in which about 80 V fragments, about 30 D fragments, and about 6 J fragments are variously assembled to encode the antigen-binding site, resulting in a total length of about 1.5 Mb (chromosome 14) for the H chain, about 2 Mb (chromosome 2) for the κL chain, and about 1 Mb (chromosome 22) for the λL chain. In order to reproduce the same variety of antibody repertoire in the other animal species as in humans, it is desirable to introduce the full length of each Ig gene; however, the DNA that can be inserted into conventional gene transfer vectors (such as plasmid, cosmid, BAC, YAC) is usually several kb to several hundred kb, and the introduction of the full length of each Ig gene has been difficult in the conventional transgenic animal preparation technique in which cloned DNA is injected into fertilized eggs.

Tomizuka et al. (Nat. Genet., 16,133-143(1997)) introduced a natural fragment (hCF) of a human chromosome carrying the Ig gene into mice (transchromosomal (TC) mice) and produced mice carrying the full-length human Ig gene. That is, first, human-mouse hybrid cells having human chromosomes with chromosome 14 containing the H-chain gene and chromosome 2 containing the κL-chain gene labeled, for example, with a drug-resistant marker are treated with a spindle fiber formation inhibitor (e.g., colcemid) for about 48 hours to prepare microcells in which one to several chromosomes or fragments thereof are encapsulated in the nuclear envelope, and the chromosomes are introduced into mouse ES cells by a micronucleate fusion method. Hybrid ES cells carrying chromosomes having the human Ig gene or fragments thereof are selected using a culture medium containing an agent and microinjected into mouse embryos in the same manner as in the preparation of conventional KO mice. From the resulting chimeric mice, germline chimeras are selected with the coat color taken as an indicator, and a TC mouse line that transmits a fragment of human chromosome 14 (TC (hCF14) ) and a TC mouse line that transmits a fragment of human chromosome 2 (TC (hCF2)) are established. KO mice (KO (IgH) and KO (IgK)) with the endogenous H-chain and κL-chain genes knocked out are prepared by a conventional method, and by repeated crosses of these four lines, a mouse line with all four genetic modifications (double TC/KO) can be established.

By applying the same method as in the case of preparing an ordinary mouse monoclonal antibody to the double TC/KO mouse prepared in the above manner, an antigen-specific human monoclonal antibody-producing hybridomas can be prepared. However, due to the instability of hCF2 containing the κL chain gene in mouse cells, there is a disadvantage in that the efficiency of hybridoma acquisition is lower than that in the case of a normal mouse.

On the other hand, the aforementioned Hu-Mab Mouse contains about 50% of the κL-chain genes, but shows the same diversity of κ chains as when it contains full-length κ chain because it has a structure with doubled variable region clusters (on the other hand, it contains only about 10% of H-chain genes, and thus, the diversity of H-chains is low and the responsiveness to antigens is insufficient); and since it is inserted into the mouse chromosome by the YAC vector (IgK-YAC), it is stably maintained in mouse cells. Taking this advantage, the TC(hCF14) mouse can be crossed with the Hu-Mab Mouse to prepare a mouse (product name: KM mouse) that stably carries hCF14 and IgK-YAC, whereby the efficiency of hybridoma acquisition and the affinity of antibody to an antigen equivalent to those of a normal mouse can be attained.

Furthermore, in order to more completely reproduce the diverse antibody repertoire in humans, human antibody-producing animals with the λL chain gene further introduced can be prepared. Such animals can be obtained by preparing a TC mouse (TC(hCF22)) with human chromosome 22 or a fragment thereof carrying the λL-chain gene introduced in the same manner as described above, and crossing it with the double TC/KO or KM mouse described above, or by constructing a human artificial chromosome (HAC) containing an H-chain gene locus and an λL-chain gene locus and introducing it into mouse cells (Nat. Biotechnol., 18, 1086-1090 (2000)).

### (iv) Preparation of a fully human antibody using a phage display human antibody library

Another approach to preparation of the fully human antibody is to use phage display. This method may cause a mutation due to PCR to be introduced into a region other than CDR, and thus there has been a few reports of HAHA production in the clinical stage; on the other hand, this method has advantages such as the absence of the risk of cross-species viral infection originating from the host animal and the infinite specificity of the antibody (antibodies against forbidden clones and sugar chains can also be prepared easily.).

Examples of the method for preparing a phage display human antibody libraries include, but are not limited to, the following.

The phage used is not particularly limited, but usually filamentous phage (Ff bacteriophage) is preferably used. One method for displaying a foreign protein on the surface of phage is to express and display it on a coat protein as a fusion protein with any of the coat proteins of g3p, g6p to g9p, but the most commonly used method is a method in which a foreign protein is fused to the N-terminus of g3p or g8p. Examples of the phage display vector include:
1) a phage display vector which has a foreign gene introduced so as to be fused to coat protein genes of the phage genome, and which has all the coat proteins displayed on the phage surface displayed as fusion proteins with the foreign protein;
2) a phage display vector which has a gene encoding a fusion protein inserted separately from a wild-type coat protein gene to have the fusion protein and the wild-type coat protein simultaneously expressed; and
3) a phage display vector which has E. coli with a phagemid vector having a gene encoding a fusion protein infected with a helper phage having a wild-type coat protein gene to produce phage particles having the fusion protein and the wild-type coat protein simultaneously expressed. In the case of 1), fusion of a large foreign protein results in loss of infectivity, and thus, types 2) or 3) are used for the preparation of an antibody library.

A specific example of the vectors is described by Holt et al. (Curr. Opin. Biotechnol., 11, 45-449 (2000)). For example, pCES1 (see J. Biol. Chem., 274, 18218-18230 (1999)) is a Fab-expressed phagemid vector that has DNA encoding a κL chain constant region downstream of the signal peptide of g3p, and a g3p coding sequence arranged via DNA encoding CH3, His-tag, c-myc tag, and an amber termination codon (TAG) downstream of the g3p signal peptide under the control of one lactose promoter. When the vector is introduced into E. coli with an amber mutation, Fab is displayed on the g3p coat protein, but when the vector is expressed in the HB2151 strain without amber mutation, etc., a soluble Fab antibody is produced. As the scFv-expressed phagemid vector, for example, pHEN1 (J.Mol. Biol., 222, 581-597 (1991)) is used.

On the other hand, examples of the helper phage include M13-K07 and VCSM13.

Another example of the phage display vector is a phage display vector in which sequences containing codons encoding cysteine are linked respectively to the 3' end of the antibody gene and the 5' end of the coat protein gene, and both of the genes are expressed separately (not as fusion proteins) at the same time such that the vector is designed to display an antibody on the coat protein of the phage surface through the S-S bond between the introduced cysteine residues (MorphoSys's CysDisplay^{™} technology).

Types of the human antibody library include naive/non-immunized libraries, synthetic libraries, and immunized libraries.

The naive/non-immune library is a library obtained by obtaining V_{H} and V_{L} genes possessed by normal humans by RT-PCR method and cloning them at random into the phage display vector described above. Usually, mRNA derived from lymphocytes in peripheral blood, bone marrow, tonsils, etc. of normal people is used as a template. In order to eliminate the bias of a V gene such as a disease history, only IgM-derived mRNAs that have not undergone class switch due to antigen sensitization are amplified, which are specifically called naive libraries. Typical examples include CAT's libraries (see J. Mol. Biol., 222, 581-597 (1991); Nat. Biotechnol., 14, 309-314 (1996)), MRC's libraries (see Annu. Rev. Immunol., 12, 433-455 (1994)), and Dyax's libraries (see J. Biol. Chem., 1999 (above); Proc. Natl. Acad. Sci. USA, 14, 7969-7974 (2000)).

The synthetic library is prepared by selecting a specific functional antibody gene in human B cells, replacing the portion of the antigen-binding region, such as CDR3, of the V gene fragment with DNA encoding a random amino acid sequence of appropriate length, and then making it into a library. Since the library can be constructed with a combination of V_{H} and V_{L} genes that originally produce functional scFv and Fab, it is considered to have excellent efficiency and stability for antibody expression. Representative examples include MorphoSys's HuCAL libraries (see J. Mol. Biol., 296, 57-86 (2000)), BioInvent's libraries (see Nat. Biotechnol., 18, 852 (2000)), Crucell's libraries (see Proc. Natl. Acad. Sci. USA, 92, 3938 (1995) ; J. Immunol. Methods, 272, 219-233 (2003)).

The immunized library is prepared by preparing mRNA from lymphocytes collected from a human with elevated blood antibody titer against a target antigen, such as patients with cancer, autoimmune diseases, infectious diseases, etc., or vaccinated individuals, or human lymphocytes artificially immunized with the target antigen by the in vitro immunization method described above in the same manner as in the case with the above naive/non-immunized library, amplifying the V_{H} and V_{L} genes by RT-PCR, and then making them into a library. Since the antibody gene of interest is originally included in the library, the antibody can be obtained even from a relatively small library.

The greater the diversity of the library, the better; realistically, however, considering the number of phages that can be handled in the following panning operation (10¹¹ to 10¹³ phages) and the number of phages required for clone isolation and amplification in normal panning (100 to 1,000 phages/clone), about 10⁸ to 10¹¹ clones are appropriate, and a library of about 10⁸ clones can usually be used in screening for antibodies with a Kd value of the order of 10⁻⁹.

The process of selecting an antibody against a target antigen by phage display method is called panning. Specifically, for example, by repeating, about 3 to 5 times, a series of operations of: contacting a phage library with a carrier with an antigen immobilized, washing off the unbound phage, then, eluting the bound phage from the carrier, and making E. coli infected with the phage for phage propagation, the phage displaying antigen-specific antibodies is enriched. Examples of the carrier for immobilizing an antigen include various carriers used in conventional antigen-antibody reactions and affinity chromatography, e.g., microplates, tubes, membranes, columns, and beads made of insoluble polysaccharides such as agarose, dextran, and cellulose, synthetic resins such as polystyrene, polyacrylamide, and silicon, or glass, metal, etc., as well as surface plasmon resonance (SPR) sensor chips. For the immobilization of the antigen, physical adsorption may be used, or a method using a chemical bond that is usually used to insolubilize and immobilize proteins or enzymes may be used. For example, the biotin-(strept)avidin system is preferably used. When an endogenous ligand that is a target antigen is a small molecule such as a peptide, special care should be taken to ensure that the portion used as the antigenic determinant is not covered with the binding to the carrier. For washing of the unbound phage, blocking solution such as BSA solution (1 to 2 times) and PBS containing surfactant such as Tween (3 to 5 times) can be used sequentially. It has been reported that the use of citrate buffer solution (pH 5) or the like is preferable. For elution of specific phage, acid (e.g., 0.1 M HCl) is usually used, but cleavage by specific proteases (for example, a gene sequence encoding a trypsin cleavage site can be introduced at the linkage between the antibody gene and the coat protein gene; in this case, since the phage to be eluted has a wild-type coat protein displayed on its surface, it can infect E. coli and proliferate even if all the coat protein is expressed as a fusion protein), competitive elution by soluble antigens, or reduction of S-S bonds (for example, in the CysDisplay^{™} described above, antigen-specific phage can be recovered by dissociating the antibody from the coat protein using an appropriate reducing agent after panning) are also possible. When the phage is eluted with acid, the eluted phage is neutralized with Tris, etc., then made to infect E. coli, cultured, and recovered by a conventional method.

Once the phages displaying the antigen-specific antibody are enriched by panning, they are made to infect E. coli and then seeded on a plate for cloning. The phages are collected again to examine their antigen-binding activity by the aforementioned antibody titer measurement assay (e.g., ELISA, RIA, FIA, etc.), FACS or SPR.

For example, in the case of using a vector with an amber termination codon introduced into the linkage between the antibody gene and the coat protein gene as a phage display vector, the phage when made to infect E. coli without the amber mutation (e.g., HB2151 strain) will cause soluble antibody molecules to be produced and secreted into the periplasm or the culture medium; thus, the isolation and purification of antibodies from the phage clones displaying the selected antigen-specific antibodies can be performed by destroying the cell wall with lysozyme, etc. to recover the extracellular fraction, and applying the same purification technique as above. Introduction of a His-tag or c-myc tag will allow an easy purification with IMAC or anti-c-myc antibody columns. In the case where cleavage by a specific protease is used for panning, the antibody molecules are separated from the phage surface by the action of the protease, and the antibody of interest can be purified by the same purification procedure accordingly. The technology for preparing fully human antibodies using human antibody-producing animals and phage-displayed human antibody libraries can also be applied to the preparation of monoclonal antibodies in other animal species. For example, an animal that is widely bred as livestock (poultry) , such as cows, pigs, sheep, goats, and chickens, or a pet animal such as dogs and cats can be taken as a target. In non-human animals, the use of immune libraries is more effective because there are fewer ethical problems with artificial immunization with target antigens.

The above is the description of a preferred embodiment of the hybridoma and monoclonal antibody production methods according to the present invention; and as described above, the present invention makes it possible to obtain an antibody that specifically binds to the conformational epitope of a protein antigen and produce a variety of monoclonal antibodies with unique specificity even in the case of conducting a booster shot.

Here, in the case of producing a monoclonal antibody against a molecule whose conformation is important, the production method according to the present invention has a very prominent advantage that a variety of clones capable of recognizing the conformational epitope can be established and a clone of interest can be selected from among them. For example, in the development of a pharmaceutical antibody targeting a membrane protein, it is extremely useful to have a method for efficiently producing antibodies that can recognize a three-dimensional structure exposed on the cell surface due to the presence of its transmembrane domain.

In addition, in the development of a pharmaceutical antibody targeting a protein whose precursor is cleaved to form a multimer expressing its function, it is desirable to obtain a monoclonal antibody recognizing the conformation of the multimer so that it does not react with the precursor but only with the multimer when formed. In addition, as a method of treating or preventing amyloidosis, which is considered to be progressed by the deposition of a protein aggregate due to conformational change of a normal protein, several monoclonal antibodies have been developed which has a poor binding ability to the monomer and can specifically recognize only the aggregate, with the mechanisms of action being elimination of aggregate, inhibition of aggregation, or suppression of aggregation. Furthermore, in Alzheimer's disease, the size of Aβ aggregates accumulated in the brain is considered to be correlated with the progression of the disease, and thus, if an antibody against a specific aggregate can be established, it could be used for development of a pharmaceutical antibody targeting a specific patient group, or for pathological diagnosis to determine the progress of the disease. It can be said that the hybridoma and monoclonal antibody production methods according to the present invention propose a very useful technology serving as resources of monoclonal antibodies that are diverse candidate materials for development of these pharmaceuticals.

### Examples

The advantageous effect of the present invention will be explained using examples and comparative examples given below. However, the technical scope of the present invention is not limited to the following examples.

### [Changes in blood antibody titer by intradermal administration of inactivated Japanese encephalitis vaccine to mice]

In this experimental example, in order to verify the utility of the present invention, which applies intradermal administration, the blood antibody titer of immunized animals with inactivated Japanese encephalitis vaccine used as an antigen was examined. Note that subcutaneous injection, which is a conventional known administration route, was employed as a control and compared with intradermal administration.

### (Antigen preparation)

Specifically, first, an antigen solution was prepared by weighing 0.075 µg, 0.3 µg, or 1.2 µg of inactivated Japanese encephalitis vaccine (JEBIK V, Mitsubishi Tanabe Pharma Corporation) and dissolving each in a predetermined amount of water for injection.

### (Initial immunization and administration method)

Four-week-old mice (ddY) were used as animals for immunization, and the antigen solutions with adjusted concentrations were administered to the back of the individuals. The mice were prepared in groups of 10 mice/group and classified into a subcutaneous treatment group and an intradermal treatment group based on the difference in antigen administration route, and further classified into groups based on the difference in the three amounts of the antigen. In the intradermal treatment group, the total volume of the solution obtained by dissolving the antigen weighed above in 20 µL of water for injection was administered. On the other hand, in the subcutaneous treatment group, the total volume of the solution obtained by dissolving the antigen weighed above in 100 µL of water for injection was administered after confirming that the tip of the needle did not touch the skin. A booster shot was performed 7 days after the initial immunization.

### (Measurement of blood antibody titer by EIA)

Serum was collected from each individual mouse 14 days after the initial immunization. Serum samples from each of the groups for the administration routes and the amounts of the antigen to be administered were added to the EIA plates with solid phase of Japanese encephalitis vaccine, and the antigen-specific IgG antibody titer was measured. The results are shown in Table 1 and FIG. 1 below. The serum samples were diluted to 160-fold and then further diluted by 2-folds, and the maximum serum dilution rate at which an absorbance value two times greater than that of the negative control is shown was calculated as the endpoint titer, as an indicator of antigen-specific antibody titer.

**[Table 1]**

| Group | Administration Route | Antigen | Antigen Amount (µg/mouse) | Mouse (Number of mice) | Antibody Titer (Endpoint Titer) |
|---|---|---|---|---|---|
| 1 | Intradermal | Dried, Cell Culture-Derived Japanese Encephalitis Vaccine | 0.075 | 10 | 20480 |
| 2 | | | 0.3 | 10 | 40960 |
| 3 | | | 1.2 | 10 | 40960 |
| 4 | Subcutaneous | | 0.075 | 10 | 0 |
| 5 | | | 0.3 | 10 | 640 |
| 6 | | | 1.2 | 10 | 1280 |

As shown in Table 1 and FIG. 1, in the intradermal treatment group, the vaccine-specific antibody titer in the blood after 14 days of the initial immunization increased to a level where no further booster shot was considered to be necessary due to the initial immunization and the booster shot 7 days later. Further, in the intradermal treatment group, the amount of antigen required to increase the specific antibody titer in the serum was much smaller than in the subcutaneous treatment group.

From these results, it was confirmed that immunogenicity of monoclonal antibodies against an antigen of interest can be enhanced not only by selecting animal species for immunization but also by selecting the administration route.

### [Selection of hybridoma producing an antibody that recognizes Aβ1-42 peptide or protofibrils]

Then, Aβ1-42 peptide and protofibrils mixed with RIBI adjuvant were intradermally administered to rats as an antigen, B-cells collected from these rats were used to prepare hybridomas, and hybridomas producing a desired antibody were selected therefrom.

### (Antigen preparation)

More specifically, first, human amyloid beta (Aβ)1-42 peptide was reacted using Beta Amyloid (1-42) Aggregation kit (purchased from rPeptide) for 18 hours at 37°C to form protofibrils. The Aβ1-42 peptide and protofibrils were then mixed with RIBI adjuvant, adjusted to have a concentration of 2 mg/mL using saline, and then used as the antigen.

### (Initial immunization and administration method)

Four-week-old rats (WKY/N) were used as animals for immunization, and Aβ1-42 peptide antigen and protofibril antigen were administered to the back of the individual in two separate locations. The rats were prepared in groups of 3 mice/group and classified into a subcutaneous treatment group and an intradermal treatment group based on the difference in antigen administration route. Note that in both groups, the administration was carried out at a dose of 20 µL/location, and in the intradermal treatment group, a wheal was confirmed in the injection site on the back of the rat. A booster shot was performed 14 and 21 days after the initial immunization.

### (Examination of blood antibody titer by EIA)

Blood samples were taken 18 and 28 days after the initial immunization, and Aβ-specific IgG antibody titer was measured by EIA from the serum of immunized individuals. More specifically, 100 ng of Aβ1-42 peptide or protofibrils was solidified on a plate, the plate was blocked with 3% skim milk, each serum sample was diluted 1000, 3000, 10000, 30000, and 100000 times, and the absorbance (OD) was measured after the addition of HRP-conjugated goat anti-rat IgG antibody. A sample prepared to have HRP-conjugated 6E10 antibody (purchased from BioLegend, Inc.) at 1 µg/mL was used as a positive control, and Native serum was used as a negative control.

### (Preparation of hybridomas)

The immunized individual with the highest blood antibody titer 28 days after the initial immunization was selected from each group. Then, a final immunization was performed 39 days after the initial immunization, and spleen and regional lymph nodes (axillary lymph node and brachial lymph node) were removed from the individuals 42 days after the initial immunization to collect and lymphocytes (B-cells) from each of the spleen and the regional lymph nodes. The collected lymphocytes (B-cells) were fused with Sp2/0 myeloma cells by electroporation to prepare hybridomas.

### (Screening by EIA)

For each combination of administration routes (the intradermal treatment group, the subcutaneous treatment group) and lymphocyte sources (spleen, the lymph nodes), positive clones reacting with Aβ1-42 peptide or protofibrils were selected by EIA from each culture supernatant having 500 hybridoma clones. In this manner, 160 positive clones in total were selected. Here, for the selected positive clones, the number of monoclonal cells producing monoclonal antibodies that react with either Aβ1-42 peptide (monomer) or the protofibrils used as antigens for the animals for immunization is summarized in Table 2 below by administration route and lymphocyte source. Table 2 below also shows the proportion of clones in each of the following three groups of: clones specific to Aβ1-42 monomer; clones specific to Aβ1-42 protofibrils; and clones that react with both of these (cross-reactive), based on the ratio of the antibody titer (Ag1) against Aβ1-42 monomer to antibody titer (Ag2) against Aβ1-42 protofibrils (Ag1/Ag2).

**[Table 2]**

| Group | Total Number of Positive Clones | Monomer-specific (Ag1/Ag2>1.5) | Proto fibril-specific (Ag1/Ag2<0.5) | Cross-reactivity (0.5<Ag1/Ag2 <1.5) |
|---|---|---|---|---|
| Intradermal, Lymph Node | 97 | 3.1% | 7.2% | 89.7% |
| Intradermal, Spleen | 5 | 0% | 0% | 100% |
| Subcutaneous, Lymph Node | 56 | 7.1% | 21.4% | 71.4% |
| Subcutaneous, Spleen | 2 | 50.0% | 0% | 50.0% |

| | | | | |
|---|---|---|---|---|
| Ag1 = Aβ1-42 monomer 100ng/well Ag2 = Aβ1-42 protofibrils 100ng/well | | | | |

From the results shown in Table 2, it can be seen that the number of positive clones obtained when using regional lymph nodes at the administration site (axillary and brachial lymph nodes in this case) as the source of lymphocytes is significantly higher than when using spleen as the source of lymphocytes (in the case of intradermal administration, 2 clones derived from spleen vs. 56 clones derived from lymph nodes). Even when the regional lymph nodes at the administration site were used as the source of lymphocytes, it is also shown that taking intradermal administration as the administration route resulted in a significantly higher proportion of the obtained positive clones specific to the protofibrils as compared with the case of the subcutaneous injection (7.2% with subcutaneous injection vs. 21.4% with intradermal administration). As such, the monoclonal antibody production method according to the present invention does not produce as many obtained positive clones as the subcutaneous treatment group (i.e., it does not improve in preparation efficiency), but has advantages that cross-reactivity can be reduced and a possibility can be increased of obtaining an antibody that bind specifically to the conformational epitope of a protein antigen, such as a monoclonal antibody specific to Aβ1-42 protofibrils having a specific conformation resulting from the aggregation of Aβ1-42 monomers.

### [Screening by Western blotting]

Antibodies were purified with a protein G column from the culture supernatant for the positive clones obtained by the EIA screening described above. On the other hand, a mixture of Aβ1-42 peptide (monomer) (4 ng/lane) and protofibrils (40 ng/lane) was applied to the gel lane for Western blotting and subjected to electrophoresis at 100 V for 120 min. Subsequently, the transfer to an Immobilon-Psq membrane was performed. Then, each monoclonal antibody prepared at a concentration of 2 µg/mL was made to react on the membrane, and the size (molecular weight) of the antigen to which each monoclonal antibody specifically binds was examined. The results are shown in Table 3 below. FIG. 2 shows a photograph showing the results of western blotting for several monoclonal antibodies.

**[Table 3]**

| Group | Total Number of Clones | Broad Band | 66 kDa | 30 kda | >230 kDa | No Band |
|---|---|---|---|---|---|---|
| Intradermal | 101 | 12.9% | 5.0% | 0.0% | 26.7% | 55.4% |
| Subcutaneous | 55 | 16.4% | 1.8% | 1.8% | 54.5% | 25.5% |

As can be seen from Table 3, among the 156 positive clones established in the present experimental example, monoclonal antibodies capable of specifically binding to >230 kDa, which is considered to be an aggregate (protofibrils) of Aβ, were found to be 26.7% of all the established monoclonal cells in the subcutaneous treatment group, whereas they were found to be 54.5% in the intradermal treatment group. Thus, according to the production method of the present invention, a broad band pattern was attained in Western blotting. Therefore, the production method according to the present invention, similarly to the results of the screening by EIA described above, delivers the advantages that cross-reactivity can be reduced and a possibility can be increased of obtaining an antibody that bind specifically to the conformational epitope of a protein antigen, such as a monoclonal antibody specific to Aβ1-42 protofibrils having a specific conformation resulting from the aggregation of Aβ1-42 monomers.

In addition, monoclonal antibody-producing cells capable of binding only to a specific multimer (30 kDa) were not confirmed to be prepared in the subcutaneous treatment group but could be established only by the production method according to the present invention. Among the 156 positive clones established in the present examples, it was only the one monoclonal antibody established by the production method of the present invention that showed binding ability only to 30 kDa.

### [Affinity of the monoclonal antibody obtained by the production method according to the present invention]

The affinity of the monoclonal antibody obtained by the monoclonal antibody production method according to the present invention described above was examined in comparison with that of the subcutaneous treatment group.

More specifically, 55 samples of monoclonal antibodies in the subcutaneous treatment group and 45 samples in the intradermal treatment group were purified with a protein G column from the culture supernatant for the positive clones obtained by the EIA screening described above.

Then, each purified monoclonal antibody was captured by an anti-rat IgG probe and read into a sensor (Octet RED 96 (ForteBio)). Then, each probe was dipped into Aβ1-42 peptide (monomer) prepared at 2000 nM or Aβ1-42 protofibrils prepared at 10 nM, and a binding constant was measured. Subsequently, each probe was dipped into PBS and a dissociation constant was measured.

As a result, the equilibrium dissociation constant KD (= k_{off}/kₒₙ) was about 10⁻⁶ to 10⁻¹² [M] in both subcutaneous and intradermal treatment groups, and it was confirmed that monoclonal antibodies with relatively high affinity were established.

### [Aβ fiber polymerization inhibitory action of the monoclonal antibody obtained by the monoclonal antibody production method according to the present invention]

It was evaluated whether the monoclonal antibody obtained by the production method according to the present invention described above has an inhibitory action on amyloid fibril polymerization of Aβ peptide.

More specifically, 14 samples of monoclonal antibodies in the subcutaneous treatment group and 16 samples in the intradermal treatment group were purified with a protein G column from the culture supernatant for the positive clones obtained by the EIA screening described above.

Next, it was evaluated whether these monoclonal antibodies had the inhibitory action on the polymerization of Aβ1-42 peptide using the Beta Amyloid (1-42) Aggregation kit (rPeptide). Aβ1-42 peptide, Thoiflavin T (ThT) , and the monoclonal antibody to be measured were prepared to have concentrations of 10 µM, 6.7 µM, and 1.0 µM, respectively. For the fluorescence intensity of ThT, absorbance (RLU) was measured at 0, 3, 24, and 28 hours by SpectraMax with an excitation wavelength of 440 nm and an emission wavelength of 485 nm, and the degree of inhibition of polymerization of Aβ1-42 peptide by each antibody was measured. Note that rat IgG antibody was added to the measurement sample as a negative control.

As a result of the above measurements, inhibition of Aβ peptide polymerization was confirmed in 4 specimens of 16 specimens for the monoclonal antibodies from monoclonal cells established in the subcutaneous treatment group, and in 3 specimens of 14 specimens for the monoclonal antibodies from monoclonal cells established in the intradermal treatment group. Determination was made using a fluorescent dye on the presence or absence of competitive inhibition on an epitope binding site for seven monoclonal antibodies that were shown to inhibit polymerization of Aβ peptide. The results are shown in Table 4 and FIG. 3 below. The vertical axis of the graph in FIG. 3 indicates the amount of light emission (Relative Light Unit; RLU) and the horizontal axis represents the elapsed time (time) . Table 4 also shows the molecular weight of the band confirmed in the Western blotting and the value of equilibrium dissociation constant (KD) .

**[Table 4]**

| Group | Clone Number | Antibody Isotype | EIA | Western Blotting | KD |
|---|---|---|---|---|---|
| Intradermal | 11G10 | IgG1 | Cross-reactive | Broad | 10⁻¹² |
| Intradermal | 13E5 | IgG2b | Cross-reactive | Broad | 10⁻¹² |
| Intradermal | 15C5 | IgG2a | Protofibril-specific | >230 kDa | 10⁻⁹ |
| Intradermal | 15D6 | IgG2a | Cross-reactive | Broad | 10⁻¹² |
| Subcutaneous | 2C6 | IgG2a | Cross-reactive | Broad | 10⁻¹⁰ |
| Subcutaneous | 2H9 | IgG2a | Cross-reactive | >230 kDa | 10⁻¹² |
| Subcutaneous | 4A9 | IgG2b | Monomer-specific | 30 KDa | 10⁻¹² |

The present application is based on Japanese Patent Application No. 2019-127110, filed on July 8, 2019, the disclosure of which is incorporated by reference in its entirety.

## Claims

1. A method for making a hybridoma producing a monoclonal antibody that specifically recognizes a conformational epitope of a protein antigen, the method comprising:
immunizing a non-human animal by intradermal administration of the protein antigen to the non-human animal;
collecting antibody-producing cells from a regional lymph node which corresponds to an administration site for the protein antigen in the non-human animal immunized by the administration of the protein antigen;
fusing the collected antibody-producing cells with myeloma cells to prepare a hybridoma population; and
selecting a specific hybridoma producing a monoclonal antibody that specifically recognizes a conformational epitope of the protein antigen from the hybridoma population.

2. The method according to claim 1, wherein the non-human animal is a mammal.

3. The method according to claim 2, wherein the non-human animal is a rodent.

4. The method according to claim 3, wherein the non-human animal is a rat, a mouse, or a rabbit.

5. The method according to claim 4, wherein the non-human animal is a rat.

6. The method according to any one of claims 1 to 5, wherein the administration site is a back or an auricle of the non-human animal.

7. The method according to any one of claims 1 to 6, wherein the regional lymph node is an axillary lymph node, a brachial lymph node, a submandibular lymph node, a parotid lymph node, or an inguinal lymph node.

8. The method according to any one of claims 1 to 7, wherein the protein antigen is an amyloid aggregate or amorphous aggregate of a protein or peptide, or a protein including a transmembrane domain.

9. The method according to any one of claims 1 to 8, wherein the protein antigen has a molecular weight of less than 6000.

10. The method according to any one of claims 1 to 9, wherein the protein antigen is administered by DNA immunization.

11. The method according to any one of claims 1 to 10, wherein intradermal administration of the protein antigen is performed multiple times.

12. The method according to any one of claims 1 to 11, wherein the protein antigen is an aggregate of amyloid β protein.

13. The method according to any one of claims 1 to 12, wherein the protein antigen is intradermally administered to the non-human animal in a form of a multimer or an aggregate.

14. The method according to any one of claims 1 to 13, wherein the protein antigen is intradermally administered to the above non-human animal in a form of recombinant cells expressing the protein antigen.

15. The method according to any one of claims 1 to 14, wherein the monoclonal antibody has an equilibrium dissociation constant of 1 × 10⁻⁷ [M] or less with respect to the conformational epitope.

16. The method according to any one of claims 1 to 15, wherein the intradermal administration is an administration only into a combined region of epidermal and dermal regions.

17. The method according to any one of claims 1 to 16, wherein the method comprises intradermally administering a solution containing the protein antigen to the non-human animal, and wherein a single dose volume of the solution is 100 µL or less per unit thickness [mm] of the skin at the administration site.

18. A hybridoma made by the method according to any one of claims 1 to 17.

19. A method for making a monoclonal antibody, comprising:
obtaining a specific hybridoma by the method for making a hybridoma according to any one of claims 1 to 17; and
obtaining a monoclonal antibody produced by the obtained specific hybridoma.

20. A monoclonal antibody specifically recognizing a conformational epitope of a protein antigen, the monoclonal antibody being made by the method for making a monoclonal antibody according to claim 19.
